# EUROPEAN PATENT SPECIFICATION

(11) **EP 2 717 850 B1**
(45) Date of publication and mention of the grant of the patent: **28.08.2019**
(21) Application number: 12727734.1
(22) Date of filing: 07.06.2012
(51) Int. Cl.: A61Q 5/12, A61K 8/41, A61K 8/42, A61K 8/34, A61K 8/06

(54) **METHOD FOR PREPARING PERSONAL CARE COMPOSITION COMPRISING MONOALKYL AMINE DUAL SURFACTANT SYSTEM AND SOLUBLE SALT**
VERFAHREN ZUR HERSTELLUNG EINER KÖRPERPFLEGEZUSAMMENSETZUNG MIT EINEM MONOALKYLAMIN-DOPPELTENSIDSYSTEM UND EINEM LÖSLICHEN SALZ
PROCÉDÉ DE PRÉPARATION D'UNE COMPOSITION DE SOINS PERSONNELS COMPRENANT UN SYSTÈME TENSIO-ACTIF DOUBLE À BASE DE MONOALKYL AMINE ET UN SEL SOLUBLE

(30) Priority: 09.06.2011 US 201161494912 P
(43) Date of publication of application: 16.04.2014
(73) Proprietor: The Procter & Gamble Company, Cincinnati, OH 45202 (US)
(72) Inventor: OKADA, Toshiyuki, Kobe Hyogo 658-0064 (JP); MAEDA, Shoko, Takatsuki Osaka 569-1127 (JP)
(74) Representative: P&G Patent Germany
(86) International application number: PCT/US2012/041191
(87) International publication number: WO 2012/170595

(56) References cited:
- WO-A2-2010/077707
- JP-A- 2005 255 627
- US-A1- 2004 116 539
- DATABASE GNPD [Online] MINTEL; 29 February 2008 (2008-02-29), "Shine Conditioner", Database accession no. 865490
- DATABASE GNPD [Online] MINTEL; 30 June 2007 (2007-06-30), "Conditioner", Database accession no. 747195

## Description

### FIELD OF THE INVENTION

The present invention relates to a method of preparing a personal care composition wherein the composition comprising a dual cationic surfactant system of a mono-alkyl amine salt and di-alkyl cationic surfactant, high melting point fatty compounds and soluble salts, and wherein the method comprising the following steps: preparing oil phase at a higher temperature; preparing an aqueous phase at a lower temperature; and mixing them in a high shear field with a rotating member. By the addition of a soluble salt, the surfactants, together with high melting point fatty compounds, can effectively transform to emulsions.

### BACKGROUND OF THE INVENTION

A variety of methods have been developed to prepare personal care composition comprising surfactants and high melting point fatty compounds and aqueous carriers.

A common preparation method for such composition is emulsification. Such emulsification is conducted by a variety of procedures, by a variety of temperatures, and by a variety of homogenizers.

For example, WO 2004/054693 discloses in Example 13, a hair conditioner prepared by the steps: preparing a water phase comprising 46.785% water, 0.3% potassium chloride and 0.1% disodium EDTA at 24-46°C; preparing an oil (emulsion) phase containing 41.785% water, 0.15% distearyl dimonium chloride, 0.84% cetrimonium chloride, and 3.0% cetyl alcohol at 65-88°C; delivering the phases through pipes which join eventually leading into a blending tube which is an antechamber section of a Sonolator®; and homogenizing the blend.

WO 2009/158441 relates to a hair conditioning composition containing a salt of stearyl amidopropyl dimethylamine and having a higher yield point. This publication discloses, at page 7, that the composition is preferably substantially free of di-long alkyl cationic surfactants in view of improved wet conditioning benefits.

WO 2010/077707 relates to a method of preparing a personal care composition by direct feeding of an oil phase and/or a water phase into a high shear field. This publication also discloses, at page 10, the composition is preferably substantially free of di-long alkyl cationic surfactants in view of improved wet conditioning benefits.

However, there remains a need for a method for preparing hair conditioning compositions and other personal care compositions which comprise di-long alkyl cationic surfactants, to effectively transform surfactants and fatty compounds to emulsions while not deteriorating wet conditioning benefit.

There may remain a need for such a method, by such effective transformation, to provide personal care compositions with, for example: (i) effective delivery of the conditioning benefits to hair and/or skin, for example, delivery of improved conditioning benefits from the same amount of active ingredients such as surfactants and fatty compounds; (ii) an improved product appearance, i.e., richer, thicker, and/or more concentrated product appearance, and which consumer may feel higher conditioning benefits from its appearance; (iii) homogeneous product appearance which is suitable as products on market; and/or (iv) rheology which is suitable as products on market and/or improved stability of such rheology.

Further, in addition to the above needs, there may exist a need for such a method which provides more flexibility of manufacturing operation and/or require less investment for high pressure.

None of the existing art provides all of the advantages and benefits of the present invention.

### SUMMARY OF THE INVENTION

The present invention is directed to a method of preparing a personal care composition,
wherein the composition comprises: a cationic surfactant system comprising a mono-alkyl amine salt cationic surfactant and a di-alkyl cationic surfactant; a high melting point fatty compound having a melting point of 25°C or higher; and an aqueous carrier,
wherein the mono-alkyl amine salt cationic surfactant is a salt of a tertiary amido amine having an alkyl group of from 12 to 22 carbon atoms; and an acid which is selected from the group consisting of L-glutamic acid, lactic acid, hydrochloric acid, malic acid, succinic acid, acetic acid, fumaric acid, tartaric acid, citric acid, L-glutamic hydrochloride, maleic acid, and mixtures thereof, at a molar ratio of the amine to the acid of from 1:0.3 to 1:2;
wherein the di-alkyl cationic surfactant is a di-alkyl quaternized ammonium salt having the following formula (I): 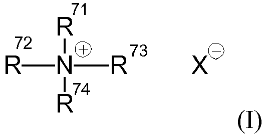 wherein two of R⁷¹, R⁷², R⁷³ and R⁷⁴ are selected from an alkyl group of from 12 to 30 carbon atoms; and the remainder of R⁷¹, R⁷², R⁷³ and R⁷⁴ are independently selected from CH₃, C₂H₅, C₂H₄OH, CH₂C₆H₅, and mixture thereof; and X⁻ is a salt-forming anion selected from the group consisting of halides such as chloride and bromide, C₁-C₄ alkyl sulfate such as methosulfate and ethosulfate, and mixtures thereof;
wherein the method comprises the steps:
(1) preparing an oil phase comprising the surfactant and the high melting point fatty compound, wherein the temperature of the oil phase is higher than a melting point of the high melting point fatty compound; and
(2) preparing an aqueous phase comprising the aqueous carrier, wherein the temperature of the aqueous phase is below the melting point of the high melting point fatty compounds; and
(3) mixing the oil phase and the aqueous phase to form an emulsion;
wherein the mixing step (3) comprises the following detailed steps:
(3-1) feeding either of the oil phase or the aqueous phase into a high shear field having an energy density of 1.0x10² J/m³ or more;
(3-2) feeding the other phase directly to the field; and
(3-3) forming an emulsion;
wherein the mixing step (3) is conducted by using a homogenizer having a rotating member; wherein the oil phase contains from 0 to 50% of the aqueous carrier by weight of the oil phase;
wherein the composition further comprises a soluble salt;
wherein the soluble salt has a solubility in water at 25°C of 0.5g/100g water or more; and
wherein the weight ratio of the soluble salt to the acid for the mono-alkyl amine salt cationic surfactant is from 1:0.5 to 1:10.

The methods of the present invention effectively transform surfactants and fatty compounds to emulsions.

These and other features, aspects, and advantages of the present invention will become better understood from a reading of the following description, and appended claims.

### DETAILED DESCRIPTION OF THE INVENTION

While the specification concludes with claims particularly pointing out and distinctly claiming the invention, it is believed that the present invention will be better understood from the following description.

Herein, "comprising" means that other steps and other ingredients which do not affect the end result can be added. This term encompasses the terms "consisting of' and "consisting essentially of'.

All percentages, parts and ratios are based upon the total weight of the compositions of the present invention, unless otherwise specified. All such weights as they pertain to listed ingredients are based on the active level and, therefore, do not include carriers or by-products that may be included in commercially available materials.

Herein, "mixtures" is meant to include a simple combination of materials and any compounds that may result from their combination.

### METHOD OF MANUFACTURING

The present invention is directed to a method of preparing a personal care composition,
wherein the composition comprises: a cationic surfactant system comprising a mono-alkyl amine salt cationic surfactant and a di-alkyl cationic surfactant; a high melting point fatty compound having a melting point of 25°C or higher; and an aqueous carrier,
wherein the mono-alkyl amine salt cationic surfactant is a salt of a tertiary amido amine having an alkyl group of from 12 to 22 carbon atoms; and an acid which is selected from the group consisting of L-glutamic acid, lactic acid, hydrochloric acid, malic acid, succinic acid, acetic acid, fumaric acid, tartaric acid, citric acid, L-glutamic hydrochloride, maleic acid, and mixtures thereof, at a molar ratio of the amine to the acid of from 1:0.3 to 1:2;
wherein the di-alkyl cationic surfactant is a di-alkyl quaternized ammonium salt having the following formula (I): 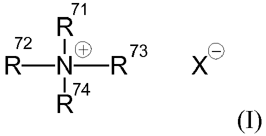 wherein two of R⁷¹, R⁷², R⁷³ and R⁷⁴ are selected from an alkyl group of from 12 to 30 carbon atoms; and the remainder of R⁷¹, R⁷², R⁷³ and R⁷⁴ are independently selected from CH₃, C₂H₅, C₂H₄OH, CH₂C₆H₅, and mixture thereof; and X⁻ is a salt-forming anion selected from the group consisting of halides such as chloride and bromide, C₁-C₄ alkyl sulfate such as methosulfate and ethosulfate, and mixtures thereof;
wherein the method comprises the steps:
(1) preparing an oil phase comprising the surfactant and the high melting point fatty compound, wherein the temperature of the oil phase is higher than a melting point of the high melting point fatty compound; and
(2) preparing an aqueous phase comprising the aqueous carrier, wherein the temperature of the aqueous phase is below the melting point of the high melting point fatty compounds; and
(3) mixing the oil phase and the aqueous phase to form an emulsion;
wherein the mixing step (3) comprises the following detailed steps:
(3-1) feeding either of the oil phase or the aqueous phase into a high shear field having an energy density of 1.0x10² J/m³ or more;
(3-2) feeding the other phase directly to the field; and
(3-3) forming an emulsion;
wherein the mixing step (3) is conducted by using a homogenizer having a rotating member;
wherein the oil phase contains from 0 to 50% of the aqueous carrier by weight of the oil phase;
wherein the composition further comprises a soluble salt;
wherein the soluble salt has a solubility in water at 25°C of 0.5g/100g water or more; and
wherein the weight ratio of the soluble salt to the acid for the mono-alkyl amine salt cationic surfactant is from 1:0.5 to 1:10.

Preferably, the method further comprises the step of adding additional ingredients such as silicone compounds, perfumes, preservatives, polymers, if included, to the emulsion. Preferably, as described below under the title "GEL MATRIX", the emulsion is a gel matrix.

### DETAILS OF MIXING STEP (3)

In the present invention, by directly feeding the phase to the high shear field, the oil phase and the aqueous phase first meet in the high shear field. It is believed that, by meeting first in the high shear field, the method of the present invention provides improved transformation of surfactants and high melting point fatty compounds to emulsions, i.e., the resulted compositions contain reduced amount of non-emulsified surfactants/high melting point fatty compounds, compared to other methods by which such phases first meet in non- or lower shear field. It is also believed that, by such improved transformation to an emulsion, the method of the present invention provides the resulted composition with improved conditioning benefits, and may also provide them with improved product appearance and/or product stability.

In the present invention, "direct feeding" means, feeding the two phases such that the two phases can reach to the high shear field after first meeting, within 0.52 seconds or less, preferably 0.5 seconds or less, more preferably 0.3 seconds or less, still more preferably 0.1 seconds or less, even more preferably 0 second, in view of improved transformation to emulsions. In the present invention, the direct feeding is preferably conducted by a direct injection.

In the present invention, "high shear field" means that the field has an energy density of from 1.0x10² J/m³, preferably from 1.0x10³ J/m³, more preferably from 1.0x10⁴ J/m³ in view of improved transformation to emulsions, and to 5.0x10⁸ J/m³, preferably to 2.0x10⁷ J/m³, more preferably to 1.0x10⁷ J/m³.

In the present invention, it is preferred that the mixing step (3) comprises the following detailed steps:
(3-1) feeding the aqueous phase into a high shear field having an energy density of 1.0x10² J/m³ or more ;
(3-2) feeding the oil phase directly to the field; and
(3-3) forming an emulsion.

In the present invention, especially when using homogenizers having a rotating member described below in detail, it is preferred to feed the oil phase into the high shear field in which the aqueous phase is already present, in view of stably manufacturing the compositions with improved conditioning benefits.

Preferably, in the present invention, the mixing step (3) including the detailed steps (3-1) and (3-2) is conducted by using a high shear homogenizer.

It is known that high shear homogenizers include, for example: high shear homogenizers having a rotating member; and high pressure homogenizers. In the present invention, high shear homogenizers having a rotating member are used, rather than high pressure homogenizers such as Sonolator ® available from Sonic Corporation, Manton Gaulin type homogenizer available from the APV Manton Corporation, and Microfluidizer available from Micro fluidics Corporation. Such a high shear homogenizer having a rotating member is believed to: provide more flexibility of manufacturing operation by its two independent operation levers (flow rate and rotating speed) while high pressure homogenizers have only one lever (pressure determined depending on flow rate); and/or require less investment for high pressure.

High shear homogenizers having a rotating member useful herein include, for example, direct injection rotor-stator homogenizers such as: Becomix ® available from A. Berents Gmbh&Co. and Lexa-30 available from Indolaval/TetraPac, in view of improved transforming to emulsions. These direct injection rotor-stator homogenizers are preferred since the two phases can quickly reach to the high shear field after first meeting, compared to other homogenizers having a rotating member, when used as-is. Such other homogenizers having a rotating member include, for example: T. K. pipeline homomixer available from Primix Corporation, and DR-3 available from IKA Corporation. Those other homogenizers having a rotating member might be used with modifications such that the two phases can quickly reach to the high shear field after first meeting. Such other homogenizers having a rotating member, when used as-is, may provide an increased amount of high melting point fatty compound crystals which are not transformed into emulsions, in the composition. Other homogenizers, which has a lower energy density, such as that named T. K. pipeline homomixer may also provide such an increased amount of high melting point fatty compound crystals

### DETAILS OF TEMPERATURE CONDITIONS

In the present invention, the oil phase has a temperature which is higher than a melting point of the high melting point fatty compounds. Preferably, the oil phase has a temperature which is higher than a melting point of the oil phase. Preferably, the oil phase has a temperature of from 25°C, more preferably from 40°C, still more preferably from 50°C, even more preferably from 55°C, further preferably from 66°C, and to 150°C, more preferably to 95°C, still more preferably to 90°C, even more preferably to 85°C, when mixing it with the aqueous phase.

In the present invention, the aqueous phase has a temperature which is below the melting point of the high melting point fatty compounds. Preferably, the aqueous phase has a temperature of from 10°C, more preferably from 15°C, still more preferably from 20°C, and to 65°C, more preferably to 55°C, still more preferably to 52°C, even more preferably to 48°C, when mixing it with the oil phase. Preferably, the temperature of the aqueous phase, when mixing it with the oil phase, is at least 5°C lower than, more preferably at least 10°C lower than the temperature of the oil phase. Preferably, the temperature of the aqueous phase, when mixing it with the oil phase, is from 2°C to 60°C lower than, more preferably from 2°C to 40°C lower than, still more preferably from 2°C to 30°C lower than the melting point of the high melting point fatty compounds.

Preferably, in the present invention, the temperature of the emulsion when formed is from 10°C to 85°C, more preferably from 25°C to 65°C. Preferably, especially when forming a gel matrix, the temperature of the emulsion when formed is from 2°C to 60°C lower than, more preferably from 2°C to 40°C lower than, still more preferably from 2°C to 30°C lower than the melting point of the high melting point fatty compounds.

### DETAILS OF OIL PHASE COMPOSITION

Oil phase comprises the surfactants and the high melting point fatty compounds. The oil phase comprises preferably from 50% to 100%, more preferably from 60% to 100%, still more preferably from 70% to 100% of the surfactants and the high melting point fatty compounds, by weight of the total amount of the surfactants and the high melting point fatty compounds used in the personal care composition, in view of providing the benefits of the present invention.

The surfactants and the high melting point fatty compounds are present in the oil phase, with or without other ingredients, at a level by weight of the oil phase of, preferably from 35% to 100%, more preferably from 50% to 100%, still more preferably from 60% to 100%, in view of providing the benefits of the present invention.

Oil phase may contain an aqueous carrier such as water and lower alkyl alcohols, and polyhydric alcohols. If included, the level of aqueous carrier in the oil phase is up to 50%, more preferably up to 40%, still more preferably up to 25%, even more preferably up to 15% by weight of the oil phase, in view of providing the benefits of the present invention. Among the aqueous carrier, it is further preferred to control the level of water in oil phase, such that the level of water in oil phase is preferably up to 40%, more preferably up to 25%, still more preferably up to 15%, even more preferably up to 10% by weight of the oil phase. The oil phase may be substantially free of water. In the present invention, "oil phase being substantially free of water" means that: the oil phase is free of water; the oil phase contains no water other than impurities of the ingredients; or, if the oil phase contains water, the level of such water is very low. In the present invention, a total level of such water in the oil phase, if included, preferably 1% or less, more preferably 0.5% or less, still more preferably 0.1% or less by weight of the oil phase.

Oil phase may contain other ingredients than the surfactants and the high melting point fatty compounds and aqueous carrier. Such other ingredients are, for example, water-insoluble components and/or heat sensitive components, such as water-insoluble silicones, water-insoluble perfumes, water-insoluble preservatives such as parabens and non-heat sensitive preservatives such as benzyl alcohol. In the present invention, "water-insoluble components added to the oil phase" means that the components have a solubility in water at 25°C of, preferably below 0.5g/100g water (excluding 0.5g/100g), more preferably 0.3g/100g water or less. If included, it is preferred that the level of such other ingredients in the oil phase is up to 50%, more preferably up to 40%, by weight of the oil phase, in view of providing the benefits of the present invention.

### DETAILS OF AQUEOUS PHASE COMPOSITION

Aqueous phase comprises aqueous carrier. The aqueous phase comprises preferably from 50% to 100%, more preferably from 70% to 100%, still more preferably from 90% to 100%, even more preferably from 95% to 100% of aqueous carrier, by weight of the total amount of the aqueous carrier used in the personal care composition, in view of providing the benefits of the present invention.

Aqueous carrier is present in the aqueous phase, with or without other ingredients, at a level by weight of the aqueous phase of, from 50% to 100%, more preferably from 70% to 100%, still more preferably from 90% to 100%, even more preferably from 95% to 100%, in view of providing the benefits of the present invention.

Aqueous phase may contain the surfactants and high melting point fatty compounds. If included, it is preferred that the level of the sum of the surfactants and high melting point fatty compounds in the aqueous phase is up to 20%, more preferably up to 10%, still more preferably up to 7% by weight of the aqueous phase, in view of providing the benefits of the present invention. Even more preferably, the aqueous phase is substantially free of the surfactants and high melting point fatty compounds. In the present invention, "aqueous phase being substantially free of the surfactants and high melting point fatty compounds" means that: the aqueous phase is free of the surfactants and high melting point fatty compounds; or, if the aqueous phase contains the surfactants and high melting point fatty compounds, the level of such surfactants and high melting point fatty compounds is very low. In the present invention, a total level of such surfactants and high melting point fatty compounds in the aqueous phase, if included, preferably 1% or less, more preferably 0.5% or less, still more preferably 0.1% or less by weight of the aqueous phase. Aqueous phase may contain other ingredients than the surfactants and the high melting point fatty compounds and aqueous carrier. Such other ingredients are, for example, the soluble salt required in the present invention, acids such as 1-glutamic acid for mono-alkyl amine salt cationic surfactants, water soluble components and/or heat sensitive components, such as water soluble pH adjusters, water soluble preservatives such as phenoxyethanol and Kathon®, and water soluble polymers. In the present invention, "water soluble components added to the aqueous phase" means that the components have a solubility in water at 25°C of, preferably at least 0.5g/100g water, more preferably at least 0.7g/100g, still more preferably at least 1.2g/100g water, further more preferably at least 1.5g/100g water, even more preferably at least 2.0g/100 water. If included, it is preferred that the level of such other ingredients in the aqueous phase is up to 20%, more preferably up to 10% by weight of the aqueous phase, in view of providing the benefits of the present invention.

### PERSONAL CARE COMPOSITION

The personal care composition of the present invention comprises a surfactant, high melting point fatty compound, and aqueous carrier. The surfactants, the high melting point fatty compounds, and the aqueous carrier are in the form of emulsion.

### CATIONIC SURFACTANT SYSTEM

The compositions of the present invention comprise a cationic surfactant system. The cationic surfactant system can be included in the composition at a level from 0.8%, preferably from 1.0%, more preferably from 1.2%, , and to 8%, preferably to 5%, more preferably to 4% by weight of the composition, in view of providing the benefits of the present invention.

Preferably, in the present invention, the surfactant is water-insoluble. In the present invention, "water-insoluble surfactants" means that the surfactants have a solubility in water at 25°C of preferably below 0.5g/100g (excluding 0.5g/100g) water, more preferably 0.3g/100g water or less.

Cationic surfactant system useful herein comprises a mono-alkyl amine salt cationic surfactant and a di-alkyl cationic surfactant. It is believed that such combination of a mono-alkyl amine salt cationic surfactant and a di-alkyl cationic surfactant provides quick rinse feel, compared to other cationic surfactant system, for example, that consisting of a mono-alkyl amine salt cationic surfactant without di-alkyl cationic surfactant, and that consisting of a mono-alkyl quaternized ammonium salt cationic surfactant which has one long alkyl chain which has from 12 to 30 carbon atoms. In the cationic surfactant system it is preferred that the weight ratio of the mono-alkyl amine salt cationic surfactant to the di-alkyl cationic surfactant is from 1:1 to 5:1., more preferably from 1.2:1 to 5:1, still more preferably from 1.5:1 to 4:1, in view of stability in rheology and conditioning benefits.

### Mono-alkyl amine salt cationic surfactant

Mono-alkyl amine salts useful herein are tertiary amido amines having an alkyl group of from 12 to 22 carbons. Exemplary tertiary amido amines include: stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, diethylaminoethylstearamide. Useful amines in the present invention are disclosed in U.S. Patent 4,275,055, Nachtigal, et al.

These amines are used in combination with ℓ-glutamic acid, lactic acid, hydrochloric acid, malic acid, succinic acid, acetic acid, fumaric acid, tartaric acid, citric acid, ℓ-glutamic hydrochloride, maleic acid, and mixtures thereof; more preferably ℓ-glutamic acid, lactic acid, citric acid, at a molar ratio of the amine to the acid of from 1 : 0.3 to 1 : 2, more preferably from 1 : 0.4 to 1 : 1.

### Di-alkyl cationic surfactant

Di-alkyl quaternized ammonium salts useful herein are those having the formula (I): 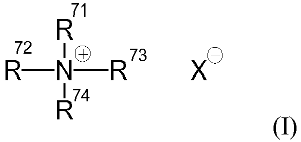 wherein two of R⁷¹, R⁷², R⁷³ and R⁷⁴ are selected from an alkyl group of from 12 to 30 carbon atoms, preferably from 16 to 24 carbon atoms, more preferably from 18 to 22 carbon atoms; the remainder of R⁷¹, R⁷², R⁷³ and R⁷⁴ are independently selected from CH₃, C₂H₅, C₂H₄OH, CH₂C₆H₅, and mixtures thereof; and X⁻ is a salt-forming anion selected from the group consisting of halides such as chloride and bromide, C1-C4 alkyl sulfate such as methosulfate and ethosulfate, and mixtures thereof. The aliphatic groups can contain, in addition to carbon and hydrogen atoms, ether linkages, and other groups such as amino groups. The longer chain aliphatic groups, e.g., those of 16 carbons, or higher, can be saturated or unsaturated.

Such preferred di-alkyl cationic surfactants include, for example, dialkyl (14-18) dimethyl ammonium chloride, ditallow alkyl dimethyl ammonium chloride, dihydrogenated tallow alkyl dimethyl ammonium chloride, distearyl dimethyl ammonium chloride, and dicetyl dimethyl ammonium chloride.

### HIGH MELTING POINT FATTY COMPOUND

The high melting point fatty compound can be included in the composition at a level of from 2%, preferably from 2.5%, more preferably from 3%, still more preferably from 35%, and to 15%, preferably to 10% by weight of the composition, in view of providing the benefits of the present invention.

The high melting point fatty compound useful herein have a melting point of 25°C or higher, preferably 40°C or higher, more preferably 45°C or higher, still more preferably 50°C or higher, in view of stability of the emulsion especially the gel matrix. Preferably, such melting point is up to 90°C, more preferably up to 80°C, still more preferably up to 70°C, even more preferably up to 65°C, in view of easier manufacturing and easier emulsification. In the present invention, the high melting point fatty compound can be used as a single compound or as a blend or mixture of at least two high melting point fatty compounds. When used as such blend or mixture, the above melting point means the melting point of the blend or mixture.

The high melting point fatty compound useful herein is selected from the group consisting of fatty alcohols, fatty acids, fatty alcohol derivatives, fatty acid derivatives, and mixtures thereof. It is understood by the artisan that the compounds disclosed in this section of the specification can in some instances fall into more than one classification, e.g., some fatty alcohol derivatives can also be classified as fatty acid derivatives. However, a given classification is not intended to be a limitation on that particular compound, but is done so for convenience of classification and nomenclature. Further, it is understood by the artisan that, depending on the number and position of double bonds, and length and position of the branches, certain compounds having certain required carbon atoms may have a melting point of less than the above preferred in the present invention. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

Among a variety of high melting point fatty compounds, fatty alcohols are preferably used in the composition of the present invention. The fatty alcohols useful herein are those having from 14 to 30 carbon atoms, preferably from 16 to 22 carbon atoms. These fatty alcohols are saturated and can be straight or branched chain alcohols.

Preferred fatty alcohols include, for example, cetyl alcohol (having a melting point of 56°C), stearyl alcohol (having a melting point of 58-59°C), behenyl alcohol (having a melting point of 71°C), and mixtures thereof. These compounds are known to have the above melting point. However, they often have lower melting points when supplied, since such supplied products are often mixtures of fatty alcohols having alkyl chain length distribution in which the main alkyl chain is cetyl, stearyl or behenyl group. In the present invention, more preferred fatty alcohols are cetyl alcohol, stearyl alcohol and mixtures thereof.

Commercially available high melting point fatty compounds useful herein include: cetyl alcohol, stearyl alcohol, and behenyl alcohol having tradenames KONOL series available from Shin Nihon Rika (Osaka, Japan), and NAA series available from NOF (Tokyo, Japan); pure behenyl alcohol having tradename 1-DOCOSANOL available from WAKO (Osaka, Japan).

### GEL MATRIX

Preferably, in the present invention, the emulsion may be in the form of a gel matrix. The gel matrix may comprise the cationic surfactant system, the high melting point fatty compound, and an aqueous carrier. The gel matrix may be suitable for providing various conditioning benefits, such as slippery feel during the application to wet hair and softness and moisturized feel on dry hair.

Preferably, especially when the gel matrix is formed, the total amount of the cationic surfactant and the high melting point fatty compound may be from 4.5%, preferably from 5.0%, more preferably from 5.5% by weight of the composition, in view of providing the benefits of the present invention, and to 15%, preferably to 14%, more preferably to 13%, still more preferably to 10% by weight of the composition, in view of spreadability and product appearance. Furthermore, when the gel matrix is formed, the cationic surfactant and the high melting point fatty compound may be contained at a level such that the weight ratio of the cationic surfactant to the high melting point fatty compound is in the range of, preferably from 1:1 to 1:10, more preferably from 1:1.5 to 1:7, still more preferably from 1:2 to 1:6, in view of providing improved wet conditioning benefits.

Preferably, when the gel matrix is formed, the composition of the present invention may be substantially free of anionic surfactants and anionic polymers, in view of stability of the gel matrix. In the present invention, "the composition being substantially free of anionic surfactants and anionic polymers" means that: the composition is free of anionic surfactants and anionic polymers; or, if the composition contains anionic surfactants and anionic polymers, the level of such anionic surfactants and anionic polymers is very low. In the present invention, a total level of such anionic surfactants and anionic polymers, if included, may be preferably 1% or less, more preferably 0.5% or less, still more preferably 0.1% or less by weight of the composition. Most preferably, the total level of such anionic surfactants and anionic polymers may be 0% by weight of the composition.

### AQUEOUS CARRIER

The composition of the present invention comprises an aqueous carrier. The level and species of the carrier are selected according to the compatibility with other components, and other desired characteristic of the product.

The carrier useful in the present invention includes water and water solutions of lower alkyl alcohols and polyhydric alcohols. The lower alkyl alcohols useful herein are monohydric alcohols having 1 to 6 carbons, more preferably ethanol and isopropanol. The polyhydric alcohols useful herein include propylene glycol, hexylene glycol, glycerin, and propane diol.

Preferably, the aqueous carrier is substantially water. Deionized water is preferably used. Water from natural sources including mineral cations can also be used, depending on the desired characteristic of the product. Generally, the compositions of the present invention comprise from 20% to 99%, preferably from 30% to 95%, and more preferably from 80% to 90% water.

### SOLUBLE SALT

The composition of the present invention further comprises a soluble salt. It has been surprisingly found by the inventors of the present invention that, when using cationic surfactant system comprising a mono-alkyl amine salt cationic surfactant and a di-alkyl cationic surfactant, the addition of the soluble salt helps effective transformation of such cationic surfactant system, together with high melting point fatty compounds, to emulsion especially gel matrix.

The soluble salt is included at a level by weight of the composition of, preferably from 0.05% to 5%, more preferably from 0.05% to 2%, still more preferably from 0.08% to 1%.

The weight ratio of the soluble salt to the acid for the mono-alkyl amine salt cationic surfactant is from 1:0.5 to 1: 10, more preferably from 1:1 to 1:8, still more preferably from 1:1.5 to 1:5.

Such soluble salts useful herein are those having a solubility in water at 25°C of preferably 0.5g/100g water or more, more preferably 1g/100g water or more, still more preferably 1.5g/100g water or more, even more preferably 3g/100g water or more.

Such soluble salts useful herein include, for example, disodium EDTA, sodium chloride, sodium citrate, sodium carbonate, and mixtures thereof.

The soluble salt can be added anytime, for example, can be added to the aqueous phase prior to the formation of emulsions, or can be added to the emulsion prior to/together with/after the addition of optional ingredients. Preferably, the soluble salt is added to the aqueous phase prior to the formation of emulsions.

### SILICONE COMPOUND

Preferably, the compositions of the present invention preferably contain a silicone compound. It is believed that the silicone compound can provide smoothness and softness on dry hair. The silicone compounds herein can be used at levels by weight of the composition of preferably from 0.1% to 20%, more preferably from 0.5% to 10%, still more preferably from 1% to 8%.

Preferably, the silicone compounds have an average particle size of from 1microns to 50 microns, in the composition.

The silicone compounds useful herein, as a single compound, as a blend or mixture of at least two silicone compounds, or as a blend or mixture of at least one silicone compound and at least one solvent, have a viscosity of preferably from 1,000 to 2,000,000mPa·s at 25°C.

The viscosity can be measured by means of a glass capillary viscometer as set forth in Dow Corning Corporate Test Method CTM0004, July 20, 1970. Suitable silicone fluids include polyalkyl siloxanes, polyaryl siloxanes, polyalkylaryl siloxanes, polyether siloxane copolymers, amino substituted silicones, quaternized silicones, and mixtures thereof. Other nonvolatile silicone compounds having conditioning properties can also be used.

Preferred polyalkyl siloxanes include, for example, polydimethylsiloxane, polydiethylsiloxane, and polymethylphenylsiloxane. Polydimethylsiloxane, which is also known as dimethicone, is especially preferred. These silicone compounds are available, for example, from the General Electric Company in their Viscasil® and TSF 451 series, and from Dow Corning in their Dow Corning SH200 series.

The above polyalkylsiloxanes are available, for example, as a mixture with silicone compounds having a lower viscosity. Such mixtures have a viscosity of preferably from 1,000mPa·s to 100,000mPa·s, more preferably from 5,000mPa·s to 50,000mPa·s. Such mixtures preferably comprise: (i) a first silicone having a viscosity of from 100,000mPa·s to 30,000,000mPa·s at 25°C, preferably from 100,000mPa·s to 20,000,000mPa·s; and (ii) a second silicone having a viscosity of from 5mPa·s to 10,000mPa·s at 25°C, preferably from 5mPa·s to 5,000mPa·s. Such mixtures useful herein include, for example, a blend of dimethicone having a viscosity of 18,000,000mPa·s and dimethicone having a viscosity of 200mPa·s available from GE Toshiba, and a blend of dimethicone having a viscosity of 18,000,000mPa·s and cyclopentasiloxane available from GE Toshiba.

The silicone compounds useful herein also include a silicone gum. The term "silicone gum", as used herein, means a polyorganosiloxane material having a viscosity at 25°C of greater than or equal to 1,000,000 centistokes. It is recognized that the silicone gums described herein can also have some overlap with the above-disclosed silicone compounds. This overlap is not intended as a limitation on any of these materials. The "silicone gums" will typically have a mass molecular weight in excess of 200,000, generally between 200,000 and 1,000,000. Specific examples include polydimethylsiloxane, poly(dimethylsiloxane methylvinylsiloxane) copolymer, poly(dimethylsiloxane diphenylsiloxane methylvinylsiloxane) copolymer and mixtures thereof. The silicone gums are available, for example, as a mixture with silicone compounds having a lower viscosity. Such mixtures useful herein include, for example, Gum/Cyclomethicone blend available from Shin-Etsu.

Silicone compounds useful herein also include amino substituted materials. Preferred aminosilicones include, for example, those which conform to the general formula (I):

(R₁)ₐG₃₋ₐ-Si-(-OSiG₂)n-( -OSiG_{b}(R₁)_{2-b})ₘ-O-SiG₃₋ₐ(R₁)ₐ

wherein G is hydrogen, phenyl, hydroxy, or C₁-C₈ alkyl, preferably methyl; a is 0 or an integer having a value from 1 to 3, preferably 1; b is 0, 1 or 2, preferably 1; n is a number from 0 to 1,999; m is an integer from 0 to 1,999; the sum of n and m is a number from 1 to 2,000; a and m are not both 0; R₁ is a monovalent radical conforming to the general formula CqH_{2q}L, wherein q is an integer having a value from 2 to 8 and L is selected from the following groups: -N(R₂)CH₂-CH₂-N(R₂)₂; -N(R₂)₂; -N(R₂)₃A ; -N(R₂)CH₂-CH₂-NR₂H₂A ; wherein R₂ is hydrogen, phenyl, benzyl, or a saturated hydrocarbon radical, preferably an alkyl radical from C₁ to C₂₀; A is a halide ion.

Highly preferred amino silicones are those corresponding to formula (I) wherein m=0, a=1, q=3, G=methyl, n is preferably from 1500 to 1700, more preferably 1600; and L is -N(CH₃)₂ or - NH₂, more preferably -NH₂. Another highly preferred amino silicones are those corresponding to formula (I) wherein m=0, a=1, q=3, G=methyl, n is preferably from 400 to 600, more preferably 500; and L is -N(CH₃)₂ or -NH₂, more preferably -NH₂. Such highly preferred amino silicones can be called as terminal aminosilicones, as one or both ends of the silicone chain are terminated by nitrogen containing group.

The above aminosilicones, when incorporated into the composition, can be mixed with solvent having a lower viscosity. Such solvents include, for example, polar or non-polar, volatile or non-volatile oils. Such oils include, for example, silicone oils, hydrocarbons, and esters. Among such a variety of solvents, preferred are those selected from the group consisting of non-polar, volatile hydrocarbons, volatile cyclic silicones, non-volatile linear silicones, and mixtures thereof. The non-volatile linear silicones useful herein are those having a viscosity of from 1 to 20,000 centistokes, preferably from 20 to 10,000 centistokes at 25°C. Among the preferred solvents, highly preferred are non-polar, volatile hydrocarbons, especially non-polar, volatile isoparaffins, in view of reducing the viscosity of the aminosilicones and providing improved hair conditioning benefits such as reduced friction on dry hair. Such mixtures have a viscosity of preferably from 1,000mPa·s to 100,000mPa·s, more preferably from 5,000mPa·s to 50,000mPa·s.

Other suitable alkylamino substituted silicone compounds include those having alkylamino substitutions as pendant groups of a silicone backbone. Highly preferred are those known as "amodimethicone". Commercially available amodimethicones useful herein include, for example, BY 16-872 available from Dow Corning.

The silicone compounds may further be incorporated in the present composition in the form of an emulsion, wherein the emulsion is made my mechanical mixing, or in the stage of synthesis through emulsion polymerization, with or without the aid of a surfactant selected from anionic surfactants, nonionic surfactants, cationic surfactants, and mixtures thereof.

### ADDITIONAL COMPONENTS

The composition of the present invention may include other additional components, which may be selected by the artisan according to the desired characteristics of the final product and which are suitable for rendering the composition more cosmetically or aesthetically acceptable or to provide them with additional usage benefits. Such other additional components generally are used individually at levels of from 0.001% to 10%, preferably up to 5% by weight of the composition.

A wide variety of other additional components can be formulated into the present compositions. These include: other conditioning agents such as hydrolysed collagen with tradename Peptein 2000 available from Hormel, vitamin E with tradename Emix-d available from Eisai, panthenol available from Roche, panthenyl ethyl ether available from Roche, hydrolysed keratin, proteins, plant extracts, and nutrients; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; coloring agents, such as any of the FD&C or D&C dyes; perfumes; ultraviolet and infrared screening and absorbing agents such as benzophenones; and antidandruff agents such as zinc pyrithione.

### PRODUCT FORMS

The compositions of the present invention can be in the form of rinse-off products or leave-on products, and can be formulated in a wide variety of product forms, including but not limited to creams, gels, emulsions, mousses and sprays. The composition of the present invention is especially suitable for hair conditioners especially rinse-off hair conditioners.

### METHOD OF USE

The composition of the present invention is preferably used for a method of conditioning hair, the method comprising following steps:
(i) after shampooing hair, applying to the hair an effective amount of the conditioning composition for conditioning the hair; and
(ii) then rinsing the hair.

Effective amount herein is, for example, from 0.1ml to 2ml per 10g of hair, preferably from 0.2 ml to 1.5ml per 10g of hair.

The composition of the present invention provides improved conditioning benefits, especially improved wet conditioning benefits after rinsing and improved dry conditioning, while maintaining wet conditioning benefit before rinsing. The composition of the present invention may also provide improved product appearance to consumer. Thus, a reduced dosage of the composition of the present invention may provide the same level of conditioning benefits as those of a full dosage of conventional conditioner compositions. Such reduced dosage herein is, for example, from 0.3ml to 0.7ml per 10g of hair.

### EXAMPLES

The following examples further describe and demonstrate embodiments within the scope of the present invention. The examples are given solely for the purpose of illustration and are not to be construed as limitations of the present invention. Where applicable, ingredients are identified by chemical or CTFA name, or otherwise defined below.

**Compositions (wt%)**

| | Components | Ex.1 | Ex.2 | Ex.3 | Ex. i | Ex. ii | Ex. iii |
|---|---|---|---|---|---|---|---|
| | Method of preparation | I | I | I | I | I | II |
| 1 | Stearamidopropyldimethylamine | 0.84 | - | 0.84 | 0.84 | 0.84 | 0.84 |
| 2 | Behenamidopropyldimethylamine | - | 1.03 | - | - | - | - |
| 3 | Varisoft 432 PPG *1 | 0.4 | 0.4 | 0.4 | 0.4 | 0.4 | 0.4 |
| 4 | Cetyl alcohol | 1.4 | 1.4 | 1.4 | 1.4 | 1.4 | 1.4 |
| 5 | Stearyl alcohol | 2.6 | 2.6 | 2.6 | 2.6 | 2.6 | 2.6 |
| 6 | Benzyl alcohol | 0.4 | 0.4 | 0.4 | 0.4 | 0.4 | 0.4 |
| 7 | Deionized Water | - | - | - | - | 7.0 | - |
| 8 | 1-Glutamic acid | 0.27 | 0.27 | 0.27 | 0.27 | 0.27 | 0.27 |
| 9 | Disodium EDTA | 0.13 | 0.13 | - | - | 0.13 | 0.13 |
| 10 | Acid EDTA | - | - | - | 0.1 | - | - |
| 11 | Water-soluble preservatives | 0.03 | 0.03 | 0.03 | 0.03 | 0.03 | 0.03 |
| 12 | Deionized Water | q.s. to 100% of the composition | | | | | |
| 13 | NaCl | - | - | 0.13 | - | - | - |
| 14 | Aminosilicone *2 | 0.8 | 1.5 | 0.8 | 0.8 | 0.8 | 0.8 |
| 15 | Perfume | 0.35 | 0.35 | 0.35 | 0.35 | 0.35 | 0.35 |
| 16 | Panthenol | 0.05 | 0.05 | 0.05 | 0.05 | 0.05 | 0.05 |
| 17 | Panthenyl ethyl ether | 0.03 | 0.03 | 0.03 | 0.03 | 0.03 | 0.03 |
| | Wet conditioning | ○ | - | ○ | × | C1 | C |

### Definitions of Components

*1 67-69% of Dicetyldimonium Chloride in q.s. to100% Propylene Glycol and 5% water, available from Evonik Goldschmidt Corporation
*2 Aminosilicone: Available from GE having a viscosity 10,000mPa·s, and having following formula (I):

   (R₁)ₐG₃₋ₐ-Si-(-OSiG₂)ₙ-(-OSiG_{b}(R₁)_{2-b})ₘ-O-SiG₃₋ₐ(R₁)ₐ (I)

   wherein G is methyl; a is an integer of 1; b is 0, 1 or 2, preferably 1; n is a number from 400 to 600; m is an integer of 0; R₁ is a monovalent radical conforming to the general formula CqH_{2q}L, wherein q is an integer of 3 and L is -NH₂

### Method of Preparation

The above hair conditioning compositions of "Ex. 1" through "Ex. 3" and "Ex. i" through "Ex. iii" were prepared by one of the following Methods I or II as shown above.

### Method I

Components 1-7 are mixed and heated to from 66°C to 85°C to form an oil phase. Separately, Components 8-12 are mixed and heated to from 20°C to 48°C to form an aqueous phase. In Becomix® direct injection rotor-stator homogenizer, the oil phase is injected and it takes 0.2 second or less for the oils phase to reach to a high shear field having an energy density of from 1.0x10⁵ J/m³ to 1.0x10⁷ J/m³ where the aqueous phase is already present. A gel matrix is formed. If included, Components 13-17 are added to the gel matrix with agitation. Then the composition is cooled down to room temperature.

### Method II

Components 1-12 are mixed with agitation, and heated to 80°C. The mixture is cooled down to 55°C and gel matrix is formed. If included, Components 13-17 are added to the gel matrix with agitation. Then the mixture is cooled down to room temperature.

### Properties and Conditioning benefits

For some of the above compositions, properties and conditioning benefits are evaluated by the following methods. Results of the evaluation are also shown above.

The embodiments disclosed and represented by "Ex. 1" through "Ex. 3" are hair conditioning compositions made by the method of the present invention which are particularly useful for rinse-off use. Such embodiments have many advantages. For example, they effectively transform to emulsions, and provides wet conditioning benefit.

Such advantages can be understood by the comparison between the examples of the present invention and comparative examples "Ex. i" through "Ex. iii". For example, improved wet conditioning benefit was observed in "Ex. 1" and "Ex. 2" of the present invention, compared to comparative examples "Ex. i" through "Ex. iii". This is because of increased rheology in "Ex. 1" and "Ex. 2" of the present invention, and because of effective transformation to lamella gel matrix in "Ex. 1" and "Ex. 2" of the present invention.

### Wet conditioning before rinsing

Wet conditioning before rinsing is evaluated by panelists, by touching a hair sample after applying 10ml compositions.
○: Perceived to provide improved wet conditioning benefit, compared to Control.
C: Control
C1: Perceived as equal to Control
× : Perceived to provide inferior wet conditioning benefit, compared to Control.

## Claims

1. A method of preparing a personal care composition,
wherein the composition comprises: a cationic surfactant system comprising a mono-alkyl amine salt cationic surfactant and a di-alkyl cationic surfactant; a high melting point fatty compound having a melting point of 25°C or higher; and an aqueous carrier,
wherein the mono-alkyl amine salt cationic surfactant is a salt of a tertiary amido amine having an alkyl group of from 12 to 22 carbon atoms; and an acid which is selected from the group consisting of L-glutamic acid, lactic acid, hydrochloric acid, malic acid, succinic acid, acetic acid, fumaric acid, tartaric acid, citric acid, L-glutamic hydrochloride, maleic acid, and mixtures thereof, at a molar ratio of the amine to the acid of from 1:0.3 to 1:2;
wherein the di-alkyl cationic surfactant is a di-alkyl quaternized ammonium salt having the following formula (I): 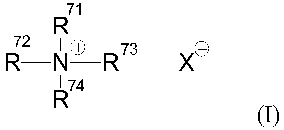 wherein two of R⁷¹, R⁷², R⁷³ and R⁷⁴ are selected from an alkyl group of from 12 to 30 carbon atoms; and the remainder of R⁷¹, R⁷², R⁷³ and R⁷⁴ are independently selected from CH₃, C₂H₅, C₂H₄OH, CH₂C₆H₅, and mixture thereof; and X⁻ is a salt-forming anion selected from the group consisting of halides such as chloride and bromide, C₁-C₄ alkyl sulfate such as methosulfate and ethosulfate, and mixtures thereof;
wherein the method comprises the steps:
(1) preparing an oil phase comprising the surfactant and the high melting point fatty compound, wherein the temperature of the oil phase is higher than a melting point of the high melting point fatty compound; and
(2) preparing an aqueous phase comprising the aqueous carrier, wherein the temperature of the aqueous phase is below the melting point of the high melting point fatty compound; and
(3) mixing the oil phase and the aqueous phase to form an emulsion;
wherein the mixing step (3) comprises the following detailed steps:
(3-1) feeding either of the oil phase or the aqueous phase into a high shear field having an energy density of 1.0x10² J/m³ or more;
(3-2) feeding the other phase directly to the field; and
(3-3) forming an emulsion;
wherein the mixing step (3) is conducted by using a homogenizer having a rotating member;
wherein the oil phase contains from 0 to 50% of the aqueous carrier by weight of the oil phase;
wherein the composition further comprises a soluble salt;
wherein the soluble salt has a solubility in water at 25°C of 0.5g/100g water or more; and
wherein the weight ratio of the soluble salt to the acid for the mono-alkyl amine salt cationic surfactant is from 1:0.5 to 1:10.

2. The method of Claim 1, wherein the mixing step (3) comprises the following detailed steps:
(3-1) feeding the aqueous phase into a high shear field having an energy density of 1.0x10² J/m³ or more ;
(3-2) feeding the oil phase directly to the field; and
(3-3) forming an emulsion.

3. The method of Claim 1, wherein the high shear field has an energy density of from 1.0x10³ J/m³.

4. The method of Claim 1, wherein the two phases reach to the high shear field within 0.52 seconds or less, after first meeting.

5. The method of Claim 1, wherein the homogenizer has a rotating member is a rotor- stator homogenizer.

6. The method of Claim 1, wherein the temperature of the emulsion is from 2°C to 60°C lower than the melting point of the high melting point fatty compound.

7. The method of Claim 1, wherein the level of water in oil phase is up to 40% by weight of the oil phase.

8. The method of Claim 1, wherein the level of water in oil phase is up to 25% by weight of the oil phase.

9. The method of Claim 1, wherein the soluble salt is selected from the group consisting of disodium EDTA, sodium chloride, sodium citrate, sodium carbonate, and mixtures thereof.

10. The method of Claim 1, wherein the soluble salt is added to the water phase.

11. A composition made by the method of Claim 1.

## Patentansprüche

1. Verfahren zum Herstellen einer Körperpflegezusammensetzung,
wobei die Zusammensetzung Folgendes umfasst: ein kationenaktives Tensidsystem, das ein kationenaktives Monoalkyl-Aminsalz-Tensid und ein kationenaktives Dialkyl-Tensid umfasst; eine Fettverbindung mit hohem Schmelzpunkt, die einen Schmelzpunkt von 25 °C oder höher aufweist; und einen wässrigen Träger,
wobei das kationenaktive Monoalkyl-Aminsalz-Tensid ein Salz eines tertiären Amidoamins ist, das eine Alkylgruppe mit 12 bis 22 Kohlenstoffatomen aufweist; und eine Säure, die ausgewählt ist aus der Gruppe bestehend aus L-Glutaminsäure, Milchsäure, Chlorwasserstoffsäure, Apfelsäure, Bernsteinsäure, Essigsäure, Fumarsäure, Weinsäure, Zitronensäure, L-Glutaminhydrochlorid, Maleinsäure und Mischungen davon, in einem Molverhältnis des Amins zu der Säure von 1:0,3 bis 1:2;
wobei das kationenaktive Dialkyl-Tensid ein quaternisiertes Dialkyl-Ammoniumsalz ist, das die folgende Formel (I) aufweist: 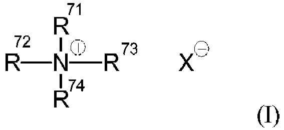 wobei zwei von R⁷¹, R⁷², R⁷³ und R⁷⁴ ausgewählt sind aus einer Alkylgruppe mit von 12 bis 30 Kohlenstoffatomen; und der Rest von R⁷¹, R⁷², R⁷³ und R⁷⁴ unabhängig voneinander ausgewählt sind aus CH₃, C₂H₅, C₂H₄OH, CH₂C₆H₅ und Mischungen davon; und X⁻ ein salzbildendes Anion ausgewählt aus der Gruppe bestehend aus Halogeniden wie Chlorid und Bromid, C₁-C₄-Alkylsulfat wie Methosulfat und Ethosulfat und Mischungen davon ist;
wobei das Verfahren die folgenden Schritte umfasst:
(1) Herstellen einer Ölphase, umfassend das Tensid und die Fettverbindung mit hohem Schmelzpunkt, wobei die Temperatur der Ölphase höher als ein Schmelzpunkt der Fettverbindung mit hohem Schmelzpunkt ist; und
(2) Herstellen einer wässrigen Phase, umfassend den wässrigen Träger, wobei die Temperatur der wässrigen Phase unter dem Schmelzpunkt dem Fettverbindung mit hohem Schmelzpunkt liegt; und
(3) Mischen der Ölphase und der wässrigen Phase, um eine Emulsion zu bilden;
wobei der Schritt des Mischens (3) die folgenden Unterschritte umfasst:
(3-1) Zuführen entweder der Ölphase oder der wässrigen Phase in ein Feld hoher Scherung mit einer Energiedichte von 1,0 x 10² J/m³ oder mehr;
(3-2) Zuführen der anderen Phase direkt zu dem Feld; und
(3-3) Bilden einer Emulsion;
wobei der Schritt des Mischens (3) unter Verwendung eines Homogenisators mit einem rotierenden Element durchgeführt wird;
wobei die Ölphase von 0 bis 50 Gew.-% der Ölphase den wässrigen Träger enthält;
wobei die Zusammensetzung ferner ein lösliches Salz umfasst;
wobei das lösliche Salz eine Löslichkeit in Wasser bei 25 °C von 0,5 g/100 g Wasser oder mehr aufweist; und
wobei das Gewichtsverhältnis des löslichen Salzes zu der Säure für das kationenaktive Monoalkyl-Aminsalz-Tensid von 1:0,5 bis 1:10 beträgt.

2. Verfahren nach Anspruch 1, wobei der Schritt des Mischens (3) ferner die folgenden Unterschritte umfasst:
(3-1) Zuführen der wässrigen Phase in ein Feld hoher Scherung mit einer Energiedichte von 1,0 x 10² J/m³ oder mehr;
(3-2) Zuführen der Ölphase direkt zu dem Feld; und
(3-3) Bilden einer Emulsion.

3. Verfahren nach Anspruch 1, wobei das Feld hoher Scherung eine Energiedichte von 1,0 x 10³ J/m³ aufweist.

4. Verfahren nach Anspruch 1, wobei die zwei Phasen nach einem ersten Treffen innerhalb von 0,52 Sekunden oder weniger zu dem Feld hoher Scherung gelangen.

5. Verfahren nach Anspruch 1, wobei der Homogenisator mit einem rotierenden Element ein Rotor-Stator-Homogenisator ist.

6. Verfahren nach Anspruch 1, wobei die Temperatur der Emulsion 2 °C bis 60 °C niedriger als der Schmelzpunkt der Fettverbindung mit hohem Schmelzpunkt ist.

7. Verfahren nach Anspruch 1, wobei der Wassergehalt in der Ölphase bis zu 40 Gew.-% der Ölphase beträgt.

8. Verfahren nach Anspruch 1, wobei der Wassergehalt in der Ölphase bis zu 25 Gew.-% der Ölphase beträgt.

9. Verfahren nach Anspruch 1, wobei das lösliche Salz ausgewählt ist aus der Gruppe bestehend aus Dinatrium-EDTA, Natriumchlorid, Natriumcitrat, Natriumcarbonat und Mischungen davon.

10. Verfahren nach Anspruch 1, wobei das lösliche Salz der Wasserphase zugesetzt wird.

11. Zusammensetzung, hergestellt durch das Verfahren nach Anspruch 1.

## Revendications

1. Procédé de préparation d'une composition de soins personnels,
dans lequel la composition comprend : un système tensioactif cationique comprenant un agent tensioactif cationique sel de mono-alkylamine et un agent tensioactif cationique di-alkyle ; un composé gras à point de fusion élevé ayant un point de fusion de 25 °C ou plus élevé ; et un véhicule aqueux,
dans lequel l'agent tensioactif cationique sel de mono-alkylamine est un sel d'une amido-amine tertiaire ayant un groupe alkyle allant de 12 à 22 atomes de carbone ; et un acide qui est choisi dans le groupe constitué d'acide L-glutamique, acide lactique, acide chlorhydrique, acide malique, acide succinique, acide acétique, acide fumarique, acide tartrique, acide citrique, chlorhydrate L-glutamique, acide maléique, et leurs mélanges, à un rapport molaire de l'amine à l'acide allant de 1:0,3 à 1:2 ;
dans lequel l'agent tensioactif cationique di-alkyle est un sel de di-alkyl ammonium quaternisé ayant la formule (I) suivante : 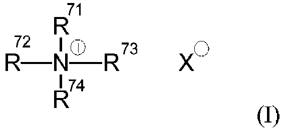 dans lequel deux parmi R⁷¹, R⁷², R⁷³ et R⁷⁴ sont choisis parmi un groupe alkyle allant de 12 à 30 atomes de carbone ; et les R⁷¹, R⁷², R⁷³ et R⁷⁴ restants sont indépendamment choisis parmi CH₃, C₂H₅, C₂H₄OH, CH₂C₆H₅, et mélange de ceux-ci ; et X⁻ est un anion salifiable choisi dans le groupe constitué d'halogénures tels que chlorure et bromure, sulfate d'alkyle en C₁ à C₄ tel que méthosulfate et éthosulfate, et mélanges de ceux-ci ;
dans lequel le procédé comprend les étapes :
(1) préparation d'une phase huileuse comprenant l'agent tensioactif et le composé gras à point de fusion élevé, dans lequel la température de la phase huileuse est supérieure à un point de fusion du composé gras à point de fusion élevé ; et
(2) préparation d'une phase aqueuse comprenant le véhicule aqueux, dans lequel la température de la phase aqueuse est inférieure au point de fusion du composé gras à point de fusion élevé ; et
(3) mélange de la phase huileuse et de la phase aqueuse pour former une émulsion ;
dans lequel l'étape de mélange (3) comprend les étapes détaillées suivantes :
(3-1) l'introduction de l'une ou l'autre de la phase huileuse ou de la phase aqueuse dans un champ à cisaillement élevé ayant une densité d'énergie de 1,0 × 10² J/m³ ou plus ;
(3-2) l'introduction de l'autre phase directement dans le champ ; et
(3-3) la formation d'une émulsion ;
dans lequel l'étape de mélange (3) est effectuée en utilisant un homogénéiseur comportant un élément rotatif ;
dans lequel la phase huileuse contient 0 à 50 % du véhicule aqueux, en poids de la phase huileuse ;
dans lequel la composition comprend en outre un sel soluble ;
dans lequel le sel soluble a une solubilité dans l'eau à 25 °C de 0,5 g/100 g d'eau ou plus ; et
dans lequel le rapport pondéral du sel soluble à l'acide pour l'agent tensioactif cationique sel de mono-alkylamine va de 1:0,5 à 1:10.

2. Procédé selon la revendication 1, dans lequel l'étape de mélange (3) comprend les étapes détaillées suivantes :
(3-1) l'introduction de la phase aqueuse dans un champ à cisaillement élevé ayant une densité d'énergie de 1,0 × 10² J/m³ ou plus ;
(3-2) l'introduction de la phase huileuse directement dans le champ ; et
(3-3) la formation d'une émulsion.

3. Procédé selon la revendication 1, dans lequel le champ à cisaillement élevé a une densité d'énergie allant de 1,0x10³ J/m³.

4. Procédé selon la revendication 1, dans lequel les deux phases arrivent au champ à cisaillement élevé en 0,52 seconde ou moins, après la première rencontre.

5. Procédé selon la revendication 1, dans lequel l'homogénéiseur comportant un élément rotatif est un homogénéiseur à rotor/stator.

6. Procédé selon la revendication 1, dans lequel la température de l'émulsion est de 2 °C à 60 °C inférieure au point de fusion du composé gras à point de fusion élevé.

7. Procédé selon la revendication 1, dans lequel le taux d'eau dans la phase huileuse vaut jusqu'à 40 % en poids de la phase huileuse.

8. Procédé selon la revendication 1, dans lequel le taux d'eau dans la phase huileuse vaut jusqu'à 25 % en poids de la phase huileuse.

9. Procédé selon la revendication 1, dans lequel le sel soluble est choisi dans le groupe constitué d'EDTA disodique, chlorure de sodium, citrate de sodium, carbonate de sodium, et mélanges de ceux-ci.

10. Procédé selon la revendication 1, dans lequel le sel soluble est ajouté à la phase aqueuse.

11. Composition fabriquée par le procédé selon la revendication 1.
